(19) Europäisches Patentamt
European Patent Office
Office européen des brevets

(11) **EP 4 183 765 A1**

(12) **EUROPEAN PATENT APPLICATION**

(43) Date of publication:
**24.05.2023 Bulletin 2023/21**

(21) Application number: **21315224.2**

(22) Date of filing: **21.11.2021**

(51) International Patent Classification (IPC):
***C07C 1/32*** *(2006.01)*     ***C07C 11/04*** *(2006.01)*
***C07C 11/06*** *(2006.01)*     ***B01J 29/70*** *(2006.01)*
***B01J 29/78*** *(2006.01)*

(52) Cooperative Patent Classification (CPC):
(C-Sets available)
**B01J 29/70; B01J 29/78; C07C 1/322;**
C07C 2529/70; C07C 2529/78            (Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **TotalEnergies OneTech
92400 Courbevoie (FR)**

(72) Inventors:
• **VERYASOV, Gleb
  1400 Nivelles (BE)**
• **NESTERENKO, Nikolai
  1402 Nivelles (Thines) (BE)**

(74) Representative: **Mellet, Valérie Martine
  Patent 42
  5, rue Dicks
  4081 Esch-sur-Alzette (LU)**

(54) **METHYL MERCAPTAN CONVERSION INTO C2-C3 OLEFINS**

(57)   The invention provides for a process for converting methyl mercaptan into C2-C3 olefins, said process comprising the steps: a) providing a gaseous feed stream comprising methyl mercaptan and optionally one or more diluents; b) providing a catalyst composition; c) contacting the gaseous feed stream with the catalyst composition at a temperature ranging from 100°C to 700°C and at a pressure ranging from 0.2 MPa to 20 MPa to convert at least part of the methyl mercaptan into C2-C3 olefins; and d) recovering said C2-C3 olefins and the unconverted methyl mercaptan if any; wherein catalyst composition comprises one or more zeolites with a plurality of pores with a shape of an 8-member ring and in that the one or more zeolites have a Si/Al atomic ratio ranging from 2 to 17 as determined by inductively coupled plasma optical emission spectrometry.

**EP 4 183 765 A1**

(52) Cooperative Patent Classification (CPC): (Cont.)

C-Sets
**C07C 1/322, C07C 11/04;**
**C07C 1/322, C07C 11/06**

**Description**

**Technical field**

The present disclosure relates to a process for converting one or more methyl mercaptans selectively into C2-C3 olefins.

**Technical background**

Olefins are used to make numerous downstream products. Starting from an alkane, and methane in particular, it is feasible to obtain the corresponding thiol alkyl, for example, methane thiol. Such thiols can be then transformed into olefins. By adjusting the reaction conditions and employing a specific catalyst, the ratios of these various olefins may be modified, leading to the obtaining of the desired effluents that can be then separated by known technology. The process for converting one or more methyl mercaptans into olefins, namely the second step of the two-step process for producing olefins from alkanes, has already been thoroughly studied.

US2007/0278136 describes a process for the production of olefins including ethylene, propylene and butenes from methyl mercaptan. The process comprises a reaction whereby methyl mercaptan decomposes to produce the olefin and hydrogen sulfide. The reaction is carried out at an elevated temperature in the range of 300° C to 500° C to achieve pyrolysis. The catalyst used is a thorium oxide catalyst or a silicon aluminium phosphorous oxide.

US4543434 describes a process for the synthesis of liquid hydrocarbon fuels and hydrogen based on the use of sulfur as an oxidant using a zeolithic catalyst. In particular, the conversion of CS2 into hydrocarbons is described.

US4822938 describes a process for converting methane to higher molecular weight hydrocarbons. In a first step, methane is contacted with elemental sulfur under conditions sufficient to produce carbon disulfide. Carbon disulfide from this step is then contacted with methane and hydrogen under conditions sufficient to convert methane and to produce $CH_3SH$. This $CH_3SH$ is then contacted with a sufficient catalyst, such as a zeolite, especially ZSM-5, under conditions sufficient to produce hydrocarbons having two or more carbon atoms.

US 2003/0060672 describes a method wherein a gas stream containing a mercaptan is passed in contact with a SAPO catalyst to convert at least a portion of the mercaptan to a hydrocarbon. M. Yu et al. (Chem. Commun., 2021,57, 3323, 10.1039/D1CC00397F) described conversion of $CH_3SH$ into light olefins on a H-SSZ-13 catalyst having a Si/Al ratio of 18.7.

The prior art documents described above show that it is possible to convert $CH_3SH$ into liquid hydrocarbons. But there is a lack of catalyst able to convert alkyl thiols into olefins mainly. The description of the prior art shows that there is a lack of catalyst composition that can efficiently direct the conversion of methyl mercaptan into olefins such as ethylene. The catalyst described are not selective toward the formation of olefins such as ethylene and propylene.

In other words, the direct conversion of one or more methyl mercaptans to C2-C3 olefins, having stable performance has not yet been described.

The present disclosure has for objective to provide a process and a catalyst wherein the conversion of one or more methyl mercaptans into olefins leads selectively to the formation of C2-C3 olefins. In particular, the present disclosure has for objective to provide a process wherein the production of olefins from one or more methyl mercaptans has an improved selectivity toward C3 olefins.

**Summary of the disclosure**

According to a first aspect, the disclosure provides a process for converting methyl mercaptan into C2-C3 olefins, said process comprising the following steps:

a) providing a gaseous feed stream comprising methyl mercaptan and optionally one or more diluents;
b) providing a catalyst composition;
c) contacting the gaseous feed stream with the catalyst composition at a temperature ranging from 100°C to 700°C and at a pressure ranging from 0.2 MPa to 20 MPa to convert at least part of the methyl mercaptan into C2-C3 olefins; and
d) recovering said C2-C3 olefins and the unconverted methyl mercaptan if any;

the process being remarkable in that said catalyst composition comprises one or more zeolites with a plurality of pores with a shape of an 8-member ring and in that the one or more zeolites have a Si/Al atomic ratio ranging from 2 to 17 as determined by inductively coupled plasma optical emission spectrometry.

Surprisingly, the inventors have found that starting from methyl mercaptan both a high selectivity to C2-C3 olefins, and in particular to propylene, and a high conversion, and subsequently a high yield, can be achieved with the use of a catalyst composition comprising one or more small-pore zeolites (i.e., having 8-member ring pores) having a Si/Al ratio of at most 17; preferably at most 15.

It has also been discovered that the introduction of olefins in the gaseous feed stream leads to an increase in the conversion and the selectivity toward C2-C3 olefins. This is can be easily be done by recycling a small part of the C2-C3 olefins produced at step c).

According to the disclosure, the C2-C3 olefins are ethylene and propylene,

In an embodiment, the one or more zeolites have a Si/Al atomic ratio ranging from 2 to 17 as determined by inductively coupled plasma optical emission spectrometry (corresponding to a SAR 4 to 34); preferably from 3 to 17; more preferably from 5 to 16; even more preferably from 8 to 15; and most preferably from 10 to 15.

In an embodiment, the one or more zeolites are selected from the group of AEI, AFX, CHA, DDR, ERI, EAB, GIS, KFI, LEV, LTA, RHO, PAU, MWF and RTH families, and any mixture thereof. For example, the one or more zeolites are selected from the group of AEI, CHA, DDR, ERI, KFI, and LEV families, and any mixture thereof. For example, the one or more zeolites are or comprise AEI and/or CHA families.

For example, the one or more zeolites are or comprise SSZ-39 from the AEI family.

For example, the one or more zeolites are or comprise SSZ-13 from the CHA family.

In an embodiment, at least one zeolite, or the one or more zeolites contain at least one metal M being a transition metal and/or a post-transition metal with an atomic ratio Si/(Al+ M) ranging from 2 to 17 as determined by inductively coupled plasma optical emission spectrometry; preferably from 3 to 17; more preferably from 5 to 16; even more preferably from 8 to 15; and most preferably from 10 to 15.

In an embodiment, at least one zeolite, or the one or more zeolites contain at least one metal M being a transition metal and/or a post-transition metal wherein the transition metal is selected from Fe, W, V, Mo, Zr, Ag, Ni, Cu, Ti, Zn and any mixture thereof and the post-transition metal is selected from Sn and/or In; preferably, the metal M is selected from Fe, W, V, Mo, Sn, Zr, Ag, Ni, Cu, Ti and any mixture thereof; more preferably, the metal M is selected from Fe, W, V, Mo, Sn, Zr, Ag and any mixture thereof; even more preferably, the metal M is selected from Fe, W, V, Mo, Sn, and any mixture thereof; most preferably, the metal M is selected from V, Mo and any mixture thereof. For example, the metal M is Mo.

In an embodiment, the one or more zeolites are present in the catalyst composition at a content ranging from 25 wt. % to 95 wt. % based on the total weight of said catalyst composition; preferably ranging from 25 to 90 wt.%; more preferably, ranging from 35 to 88 wt.%; even more preferably ranging from 40 to 85 wt.%; most preferably ranging from 50 to 82 wt.%; even most preferably ranging from 60 to 80 wt.%; for example, ranging from 70 to 85 wt.% or ranging from 50 to 90 wt.%.

In an embodiment, the catalyst composition further comprises a binder. With preference, one or more of the following can be used to further define the binder:

- The binder is selected from silica, clays, alumina phosphates, calcium phosphates, magnesium phosphates, mullite and any mixture thereof.
- The binder is or comprises silica.
- The binder is present in an amount of at least 5 wt.% or of at least 10 wt.% as based on the total weight of the catalyst composition, preferably of at least 20 wt.%, most preferably of at least 30 wt.%, even more preferably of at least 40 wt.% and most preferably of at least 50 wt.%.
- The binder is present in an amount ranging from 5 wt.% to 75 wt.% based on the total weight of said catalyst composition; preferably ranging from 10 to 75 wt.%; more preferably, ranging from 12 to 65 wt.%; even more preferably ranging from 15 to 60 wt.%; most preferably ranging from 18 to 50 wt.%; even most preferably ranging from 20 to 40 wt.%; for example, ranging from 15 to 30 wt.% or ranging from 10 to 50 wt.%.

For example, the process further comprises a step of activation of the catalyst composition that is performed before said step c) of contacting the gaseous feed stream. For example, the step of activation comprises calcinating the catalyst composition; with preference, the catalyst composition is calcined at a temperature ranging from 400°C 800°C for a period ranging from 1 h to 24h.

In an embodiment, methyl mercaptan is present in the gaseous feed stream at a concentration of at least 10 vol % based on the total volume content of the gaseous feed stream; preferably, of at least 25 vol %; most preferably of at least 35 vol%.

In an embodiment, methyl mercaptan is present in the gaseous feed stream at a concentration of 100 vol % based on the total volume content of the gaseous feed stream; preferably, of at most 95 vol %; most preferably of at most 75 vol%.

In an embodiment, the gaseous feed stream comprises one or more diluents, wherein the one or more diluents are selected from hydrogen sulphide, steam, methane, ethane, propane, butane, carbon dioxide, carbon monoxide, nitrogen, and any mixture thereof. For example, the one or more diluents are selected from steam, methane, ethane, propane, butane, carbon dioxide, carbon monoxide, nitrogen, and any mixture thereof.

In an embodiment, the gaseous feed stream comprises one or more selected from methanethiol, $CH_3SH$; ethanethiol, $C_2H_5SH$; 1-propanethiol, $C_3H_7SH$; 2-propanethiol, $CH_3CH(SH)CH_3$; allyl mercaptan; $CH_2=CHCH_2SH$, butanethiol, $C_4H_9SH$; *tert*-butyl mercaptan, $(CH_3)_3CSH$; pentanethiols, $C_5H_{11}SH$; or any mixture thereof.

In an embodiment, the one or more diluents are present in the gaseous feed stream at a concentration of at most 90 vol % based on the total volume content of the gaseous feed stream; preferably, of at most 75 vol %; most preferably of at most 65 vol%.

In an embodiment, the process further comprises a step of recycling a part of the C2-C3 olefins recovered in the gaseous feed stream. For example, the content of the recycled C2-C3 olefins in the gaseous feed stream is at most 10 vol.% based on the total volume of the gaseous feed stream; preferably, at most 8 vol.%; more preferably at most 5 vol.% and even more preferably at most 3 vol.%.

For example, the content of the recycled C2-C3 olefins in the gaseous feed stream is at least 0.5 vol.% based on the total volume of the gaseous feed stream; preferably, at least 1.0 vol.%; more preferably at least 1.5 vol.% and even more preferably at least 2.0 vol.%.

For example, step c) is performed at a temperature ranging from 200°C to 600°C; preferably ranging from 250°C to 550°C, more preferably, ranging from 300°C to 500°C; even more preferably from 320 °C to 480°C; most preferably to 350 °C to 450°C; and even most preferably from 370°C to 420°C.

For example, step c) is performed in the absence of $H_2$.

In an embodiment, the process further comprises a step of recycling the unconverted methyl mercaptan back into step c).

In an embodiment, step c) of contacting the gaseous feed stream with the catalyst composition is followed by a step d) of recovering an effluent comprising C2-C3 olefins, unreacted methyl mercaptan, alkane and higher hydrocarbons and optionally said diluent; wherein the selectivity to C3 olefin is of at least 35 %; preferably, of at least 40 %; more preferably, of at least 45 %.

According to a second aspect, the disclosure provides for an installation to conduct the process for converting methyl mercaptan into C2-C3 olefins according to the process of the first aspect, the installation is remarkable in that it comprises:

- a reactor comprising a catalyst composition, the reactor having an inlet and an outlet, wherein the catalyst composition comprises one or more zeolites with a plurality of pores with a shape of an 8-member ring and a Si/Al atomic ratio ranging from 2 to 17 as determined by inductively coupled plasma optical emission spectrometry;
- a line to convey a gaseous feed stream comprising methyl mercaptan to the inlet of the reactor;
- a first separation unit placed at the outlet of the reactor and configured to separate the unreacted methyl mercaptan and a line to recycle the unreacted methyl mercaptan at the inlet of the reactor;
- optionally, a second separation unit to recover a part of the olefins produced the reactor and a line to recycle part of the olefins recovered at the inlet of the reactor.

According to a third aspect, the disclosure provides for the use of a catalyst composition in a process for converting methyl mercaptan into C2-C3 olefins; remarkable in that the catalyst composition comprises one or more zeolites with a plurality of pores with a shape of an 8-member ring and in that the one or more zeolites have a Si/Al atomic ratio ranging from 2 to 17 as determined by inductively coupled plasma optical emission spectrometry; with preference, the process is according to the first aspect.

## Description of the figures

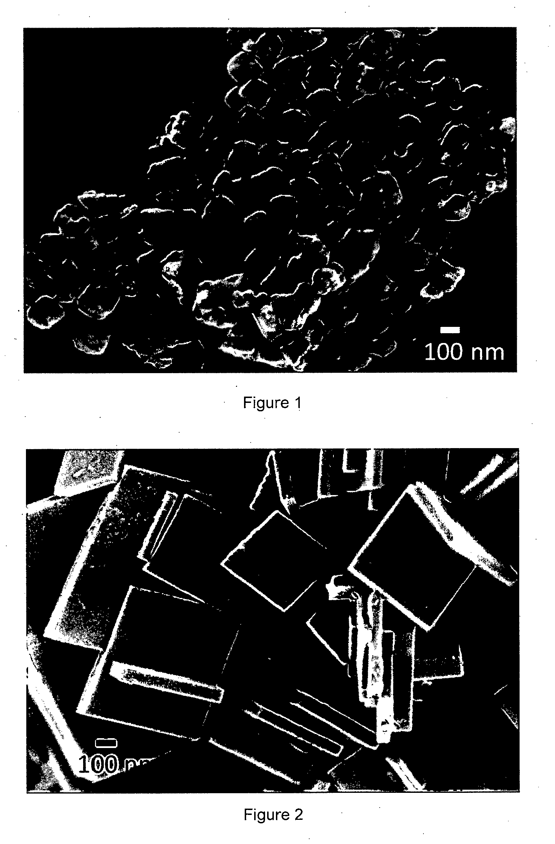

- Figure 1 is a SEM image of SSZ-13 zeolite (CHA family).
- Figure 2 is a SEM image of SSZ-39 zeolite (AEI family).

## Definitions

For the purpose of the disclosure, the following definitions are given:
Zeolite codes (e.g., CHA...) are defined according to the "Atlas of Zeolite Framework Types", 6th revised edition, 2007, Elsevier, to which the present application also refers.

The terms "alkane" or "alkanes" as used herein describe acyclic branched or unbranched hydrocarbons having the general formula $C_nH_{2n+2}$, and therefore consisting entirely of hydrogen atoms and saturated carbon atoms; see e.g. IUPAC. Compendium of Chemical Terminology, 2nd ed. (1997). The term "alkanes" accordingly describes unbranched alkanes ("normal-paraffins" or "n-paraffins" or "n-alkanes") and branched alkanes ("iso-paraffins" or "iso-alkanes") but excludes naphthenes (cycloalkanes).

The term "aromatic hydrocarbons" or "aromatics" relates to cyclically conjugated hydrocarbon with a stability (due to derealization) that is significantly greater than that of a hypothetical localized structure (e.g. Kekule structure). The most common method for determining aromaticity of a given hydrocarbon is the observation of diatropicity in the $^1$H NMR spectrum.

The terms "olefin" or "alkene" as used herein relate to an unsaturated hydrocarbon compound containing at least one carbon-carbon double bond.

The terms "mono-olefin" as used herein relates to an unsaturated hydrocarbon compound containing one single carbon-carbon double bond.

The Si/Al atomic ratio corresponds to the amount of $SiO_2$ divided by the amount of $Al_2O_3$ taking into account the fact there are two atoms of aluminium for one atom of silicon. The silica to alumina ratio (also stated as SAR) corresponds to the amount of $SiO_2$ divided by the amount of $Al_2O_3$ notwithstanding the proportion of the Si atoms over the Al atoms in the chemical formula of the molecular sieve. Therefore, the value of the SAR always corresponds to twice the value of the Si/Al atomic ratioSAR is determined by $NH_3$-Temperature Programmed Desorption.

As used herein, the term "C# hydrocarbons", wherein "#" is a positive integer, is meant to describe all hydrocarbons having # carbon atoms. C# hydrocarbons are sometimes indicated as just C#. Moreover, the term "C#+ hydrocarbons" is meant to describe all hydrocarbon molecules having # or more carbon atoms.

The symbol "=" in the term "C#= hydrocarbon" indicates that the hydrocarbon concerned is an olefin or an alkene, the notation "=" symbolizing the carbon-carbon double bond. For instance, "C6=" stands for "C6 olefin", or for "olefins comprising 6 carbon atoms".

The term "steam" is used to refer to water in the gas phase, which is formed when water boils.

The term "alkali metal" refers to an element classified as an element from the group 1 of the periodic table of elements, excluding hydrogen. According to this definition, the alkali metals are Li, Na, K, Rb, Cs and Fr.

The term "alkaline earth metal" refers to an element classified as an element from the group 2 of the periodic table of elements. According to this definition, the alkaline earth metals are Be, Mg, Ca, Sr, Ba and Ra.

The term "transition metal" refers to an element whose atom has a partially filled d sub-shell, or which can give rise to cations with an incomplete d sub-shell (IUPAC definition). According to this definition, the transition metals are Sc, Ti, V, Cr, Mn, Fe, Co, Ni, Cu, Zn, Y, Zr, Nb, Mo, Tc, Ru, Rh, Pd, Ag, Cd, La, Hf, Ta, W, Re, Os, Ir, Pt, Au, Hg, Ac, Rf, Db, Sg, Bh, Hs, Mt, Ds, Rg, and Cn. The metals Ga, In, Sn, Tl, Pb and Bi are considered as "post-transition" metal.

The yield to particular chemical compounds is determined as the mathematical product between the selectivity to said particular chemical compounds and the conversion rate of the chemical reaction. The mathematical product is expressed as a percentage.

The terms "comprising", "comprises" and "comprised of" as used herein are synonymous with "including", "includes" or "containing", "contains", and are inclusive or open-ended and do not exclude additional, non-recited members, elements or method steps. The terms "comprising", "comprises" and "comprised of" also include the term "consisting of".

The recitation of numerical ranges by endpoints includes all integer numbers and, where appropriate, fractions subsumed within that range (e.g. 1 to 5 can include 1, 2, 3, 4, 5 when referring to, for example, a number of elements, and can also include 1.5, 2, 2.75 and 3.80, when referring to, for example, measurements). The recitation of endpoints also includes the recited endpoint values themselves (e.g. from 1.0 to 5.0 includes both 1.0 and 5.0). Any numerical range recited herein is intended to include all sub-ranges subsumed therein.

The particular features, structures, characteristics or embodiments may be combined in any suitable manner, as would be apparent to a person skilled in the art from this disclosure, in one or more embodiments.

## Detailed description

The disclosure provides for a process and an installation for converting methyl mercaptan into C2-C3 olefins wherein an improved selectivity to propylene is obtained.

According to the disclosure, the process for converting methyl mercaptan into C2-C3 olefins, said process comprising the following steps:

    a) providing a gaseous feed stream comprising methyl mercaptan and optionally one or more diluents;
    b) providing a catalyst composition;
    c) contacting the gaseous feed stream with the catalyst composition at a temperature ranging from 100°C to 700°C, at a pressure ranging from 0.2 MPa to 20 MPa, to convert at least part of the methyl mercaptan into C2-C3 olefins; and
    d) recovering said C2-C3 olefins and the unconverted methyl mercaptan if any;

wherein the catalyst composition comprises one or more zeolites with a plurality of pores with a shape of an 8-member ring and in that the one or more zeolites have a Si/Al atomic ratio ranging from 2 to 17 as determined by inductively coupled plasma optical emission spectrometry.

In the present description, the installation will be described simultaneously with the process. The installation to conduct the process for converting methyl mercaptan into C2-C3 olefins according to the process of the first aspect, the installation is remarkable in that it comprises:

- a reactor comprising a catalyst composition, the reactor having an inlet and an outlet, wherein the catalyst composition comprises one or more zeolites with a plurality of pores with a shape of an 8-member ring and a Si/Al atomic ratio ranging from 2 to 17 as determined by inductively coupled plasma optical emission spectrometry;
- a line to convey a gaseous feed stream comprising methyl mercaptan to the inlet of the reactor;
- a first separation unit placed at the outlet of the reactor and configured to separate the unreacted methyl mercaptan and a line to recycle the unreacted methyl mercaptan at the inlet of the reactor;
- optionally, a second separation unit to recover a part of the olefins produced the reactor and a line to recycle part of the olefins recovered at the inlet of the reactor.

For example, step c) is performed in fixed bed or in fluidized bed; thus, the reactor in the installation is a fixed bed reactor or a fluidized bed reactor.

The composition of the gaseous feed stream

The gaseous feed stream of step a) contains methyl mercaptan. Any type of methyl mercaptan can be used.
Preferably, methyl mercaptan is present in the gaseous feed stream at a concentration of at least 10 vol % based on the total volume content of the gaseous feed stream; preferably, of at least 20 vol %; more preferably, of at least 25 vol %; most preferably of at least 35 vol%.
In an embodiment, methyl mercaptan is present in the gaseous feed stream at a concentration of 100 vol % based on the total volume content of the gaseous feed stream; preferably, of at most 95 vol %; most preferably of at most 75 vol%.
In a most preferred embodiment, the one or more diluents are or comprise methane. With preference, methane is present in said the gaseous feed stream at a concentration ranging from 10 vol % to 90 vol %, preferably at a concentration ranging from 25 vol % to 75 vol %, even more preferably at a concentration ranging from 33 vol % to 66 vol %.
In an embodiment, the one or more diluents comprise $H_2S$ at a concentration of at least 1 vol % to at most 10 vol %. In an embodiment, the gaseous feed stream comprises one or more selected from selected from methanethiol, $CH_3SH$; ethanethiol, $C_2H_5SH$; 1-propanethiol, $C_3H_7SH$; 2-propanethiol, $CH_3CH(SH)CH_3$; allyl mercaptan; $CH_2=CHCH_2SH$, butanethiol, $C_4H_9SH$; *tert*-butyl mercaptan, $(CH_3)_3CSH$; pentanethiols, $C_5H_{11}SH$; or any mixture thereof.
In a preferred embodiment, the one or more diluent are present in said stream a) at a concentration ranging from 10 mol % to 90 mol %, preferably at a concentration ranging from 25 mol % to 75 mol %, even more preferably at a concentration ranging from 33 mol % to 66 mol %.
In a most preferred embodiment, the diluent is $CH_4$, present in said stream a) at a concentration ranging from 10 mol % to 90 mol %, preferably at a concentration ranging from 25 mol % to 75 mol %, even more preferably at a concentration ranging from 33 mol % to 66 mol %.
In a preferred embodiment, the gaseous feed stream of step a) further comprises other compounds such as $H_2S$ at a concentration of at least 1 mol % to at most 10 mol %. For example, the content of $H_2S$ in the gaseous feed stream is less than 5 vol% based on the volume total of the gaseous feed stream.

The operating conditions of step c)

In principle, the reaction temperature is between 100 and 700°C, preferably between 270 and 600°C, most preferably between 280 and 550°C. The pressure is between 0.1 and 5.0 MPa, preferably between 0.15 and 3.0 MPa, even more preferably between 0.2 and 2.0 MPa.

The catalyst composition of the present disclosure

According to the disclosure, the catalyst composition comprises one or more zeolites with a plurality of pores with a shape of 8-member ring and having a Si/Al atomic ratio ranging from 2 to 17 as determined by inductively coupled plasma optical emission spectrometry.
This requirement of having small pores can be understood in that the largest pore has a shape of 8-member ring. For example, a chabazite-type zeolite comprises at least two cages composed of 4- and 8- membered rings connected by one 6-membered double ring.

The requirement of Si/Al ratio to be low is to control the acidity of the one or more zeolites.

With regards to the catalyst composition, it can comprise a fairly wide range of zeolites. However, in an embodiment, the one or more zeolites are selected from the group of AEI, AFX, CHA, DDR, ERI, EAB, GIS, KFI, LEV, LTA, RHO, PAU, MWF and RTH families, and any mixture thereof. For example, the one or more zeolites are selected from the group of AEI, CHA, DDR, ERI, KFI, and LEV families, and any mixture thereof. For example, the one or more zeolites are or comprise AEI and/or CHA families.

For example, the one or more zeolites are or comprise SSZ-39 from the AEI family. The synthesis of SSZ-39 is for example disclosed in US10399858.

For example, the one or more zeolites are or comprise SSZ-13 from the CHA family. SSZ-13 zeolites are known to the person skilled in the art. The first synthesis of aluminosilicate CHA zeolite (SSZ-13) has been performed by S.I. Zones in 1985 (see US patent application 4,544,538) using organic-structure-directing agents *N,N,N*-trimethyladamantammonium hydroxide (TMAdaOH) coupled with OH$^-$ as a mineralizing agent.

The zeolites can be prepared using an Organic Structure Directing Agents (OSDAs) or without. In some cases, the precursor zeolites contain alkali (e.g., Na$^+$, K$^+$, Cs$^+$), alkaline earth, or transition metal cations used, for example, in the preparation of the precursor zeolite. In some embodiments, at least a portion of these cations are still present in as-prepared zeolite.

In some embodiments, the crystalline aluminosilicate compositions of the present disclosure contain an 8-membered ring zeolite structure, and are independently featured by two or more of:

(a) an atomic ratio of tetrahedral to total aluminium atoms in a range having a lower value of about 0.1, 0.12, 0.14, 0.16, 0.18, 0.2, or 0.25, and having an upper value of about 0.2, 0.3, 0.4, 0.5, or 0.6, preferably from about 0.1 to about 0.5, more preferably from about 0.12 to about 0.2;

(b) a microporous region and a mesoporous region, in which the microporous region comprises tetrahedral aluminium and the mesoporous region contains tetrahedral, pentacoordinate, and/or octahedral (hexacoordinate) aluminium;

(c) a micropore volume comprising from 0.03 to 0.15, preferably 0.03 to 0.8, mL/g of the composition.

With regards to the catalyst composition, it can comprise a fairly wide range of zeolites. In certain embodiments, a portion of the aluminium in the zeolite framework may be substituted with a transition metal, boron, gallium, and/or titanium. In certain embodiments, a portion of the silicon in the zeolite framework may be optionally substituted with phosphorus. In certain embodiments, the one or more zeolites could contain tin, molybdenum, tungsten, or iron in the tetrahedral sites of the framework. The zeolites generally may have a significant anionic charge within the zeolites framework structure which may be balanced, for example, by cations of elements selected from the group H, Li, Na, K or Cs or the group Mg, Ca, Sr or Ba or the group La or Ce. Although zeolitic catalysts may be commonly obtained in a sodium form, a protonic or hydrogen form (via ion-exchange with ammonium hydroxide, and subsequent calcining) is preferred, or a mixed protonic/sodium form may also be used. The one or more zeolites may also be modified by ion-exchange with alkali metal cations, such as Li, K, or Cs, with alkali-earth metal cations, such as Mg, Ca, Sr, or Ba, or with transition metal cations, such as Fe, Ni, Cu, Mn, V, W or with rare-earth metal cations, such as La or Ce. Such subsequent ion-exchange may replace the charge-balancing counter-ions, but furthermore may also partially replace ions in the oxide framework resulting in a modification of the crystalline make-up and structure of the oxide framework.

In an embodiment, the one or more zeolites have a Si/Al atomic ratio ranging from 2 to 17 as determined by inductively coupled plasma optical emission spectrometry (corresponding to a SAR 4 to 34); preferably from 3 to 17; more preferably from 5 to 16; even more preferably from 8 to 15; and most preferably from 10 to 15.

For example, the one or more zeolites have a Si/Al atomic ratio of at most 17 as determined by inductively coupled plasma optical emission spectrometry; preferably at most 16; more preferably, of at most 25.

For example, the one or more zeolites have a Si/Al atomic ratio of at least 2 as determined by inductively coupled plasma optical emission spectrometry; preferably at least 5; more preferably, of at least 8; even more preferably, of at least 10; most preferably, of at least 11; or of at least 12.

In an embodiment, at least one zeolite, or the one or more zeolites contain at least one metal M being a transition metal and/or a post-transition metal with an atomic ratio Si/(Al+ M) ranging from 2 to 17 as determined by inductively coupled plasma optical emission spectrometry; preferably from 3 to 17; more preferably from 5 to 16; even more preferably from 8 to 15; and most preferably from 10 to 15.

For example, at least one zeolite, or the one or more zeolites contain at least one metal M being a transition metal and/or a post-transition metal with an atomic ratio Si/(Al+ M) of at most 25 as determined by inductively coupled plasma optical emission spectrometry; preferably at most 22; more preferably, of at most 20; even more preferably, of at most 18; most preferably, of at most 17; or of at most 16 or of at most 15.

For example, at least one zeolite, or the one or more zeolites contain at least one metal M being a transition metal and/or a post-transition metal with an atomic ratio Si/(Al+ M) of at least 2 as determined by inductively coupled plasma optical emission spectrometry; preferably at least 5; more preferably, of at least 8; even more preferably, of at least 10; most preferably, of at least 11; or of at least 12.

In an embodiment, at least one zeolite, or the one or more zeolites contain at least one metal M being a transition metal and/or a post-transition metal wherein the transition metal is selected from Fe, W, V, Mo, Zr, Ag, Ni, Cu, Ti, Zn and any mixture thereof and the post-transition metal is selected from Sn and/or In; preferably, the metal M is selected from Fe, W, V, Mo, Sn, Zr, Ag, Ni, Cu, Ti and any mixture thereof; more preferably, the metal M is selected from Fe, W, V, Mo, Sn, Zr, Ag and any mixture thereof; even more preferably, the metal M is selected from Fe, W, V, Mo, Sn, and any mixture thereof; most preferably, the metal M is selected from V, Mo and any mixture thereof. For example, the metal M is Mo.

For example, the one or more zeolites comprise a content ranging from 0.01 to 10.0 wt.% of metal M based on the total mass of the zeolite as measured according to EDS-TEM; preferably a content ranging from 0.1 to 5.0 wt.% more preferably ranging from 0.1 to 1.5 wt.%.

Isomorphous substitution is well known to the person skilled in the art.

Traditionally, post-synthesis incorporation of heteroelements (for example, a metal M) can be achieved by the treatment of a pre-formed zeolite with a volatile source of a heteroelement at elevated temperature (gas phase isomorphous substitution) or with a solution of the heteroelement source at moderate temperature (hydrothermal isomorphous substitution). Moreover, solid-state reaction can be applied for tailoring the chemical composition of zeolites.

The removal of some framework atoms leading to the formation of silanol groups (Si-OH) usually precedes the insertion of heteroelements. Dealumination, routinely performed by steaming or treatment of zeolites with mineral acids or the combination of both methods, is one of the examples for demetallation treatments.

In contrast, desilication in alkaline medium successfully applied to a large variety of zeolites does not cause significant changes in the Si/Al ratio and, therefore, in acidity. The desilication procedure clearly allows developing mesoporosity in zeolites but quite often at the expense of losing considerable amounts of microporosity. Different variations of the method have been introduced to overpass this drawback, such as the substitution of sodium hydroxide for tetraalkylammonium hydroxide solutions or the application of partial detemplation prior to desilication.

In an embodiment, the one or more zeolites are present in the catalyst composition at a content ranging from 25 wt. % to 95 wt. % based on the total weight of said catalyst composition; preferably ranging from 25 to 90 wt.%; more preferably, ranging from 35 to 88 wt.%; even more preferably ranging from 40 to 85 wt.%; most preferably ranging from 50 to 82 wt.%; even most preferably ranging from 60 to 80 wt.%; for example, ranging from 70 to 85 wt.% or ranging from 50 to 90 wt.%.

The binder is preferably selected from silica, clays, alumina phosphates, calcium phosphates, magnesium phosphates, mullite and any mixture thereof. With preference, the binder is or comprises silica.

The binder is preferably present in an amount of at least 5 wt.% or of at least 10 wt.% as based on the total weight of the catalyst composition, preferably of at least 20 wt.%, most preferably of at least 30 wt.%, even more preferably of at least 40 wt.% and most preferably of at least 50 wt.%.

The binder is preferably present in an amount ranging from 5 wt.% to 75 wt.% based on the total weight of said catalyst composition; preferably ranging from 10 to 75 wt.%; more preferably, ranging from 12 to 65 wt.%; even more preferably ranging from 15 to 60 wt.%; most preferably ranging from 18 to 50 wt.%; even most preferably ranging from 20 to 40 wt.%; for example, ranging from 15 to 30 wt.% or ranging from 10 to 50 wt.%.

For example, the one or more zeolites are shaped with a binder, which is an inorganic material, and preferentially silica or alumina. The zeolites shaped with the binder forms a catalyst composition, and the catalyst composition of the present disclosure preferably comprises at least 5 wt.% of a binder, at most 40 wt.% as based on the total weight of the catalyst composition and at most 40 wt.%. Typically, the catalyst composition of the present disclosure comprises between 20 wt.% and 25 wt.% to at most 80 wt. % of a binder as based on the total weight of the catalyst composition.

Non-limiting examples of silicon sources suitable for the binder of the catalyst composition include silicates, precipitated silicas, for example, Zeosil® available from Rhodia, fumed silicas, for example, Aerosil®200 available from Degussa Inc., New York, N.Y., silicon compounds such as tetraalkyl orthosilicates, for example, tetramethyl orthosilicate (TMOS) and tetraethylorthosilicate (TEOS), colloidal silicas or aqueous suspensions thereof, for example Ludox® HS-40 available from E.I. du Pont de Nemours, Wilmington, Del., silicic acid, alkali-metal silicate, or any combination thereof.

Other suitable forms of amorphous silica include silica powders, such as Ultrasil® VN3 SP (commercially available from Degussa).

Other non-limiting examples of a suitable solid silica source are special granulated hydrophilic fumed silicas, mesoporous silica and high surface area precipitated silica SIPERNAT® from Evonik, Hi-Sil 233 EP (available from PPG Industries) and Tokusil (available from Tokuyama Asia Pacific).

In addition, suitable amorphous silica sources include silica sols, which are stable colloidal dispersions of amorphous silica particles in an aqueous or organic liquid medium, preferably water.

Non-limiting examples of commercially available silica sols include those sold under the tradenames Nyacol®

(available from Nyacol Nano Technologies, Inc. or PQ Corp.), Nalco (available from Nalco Chemical Company), Ultra-Sol (available from RESI Inc), Ludox® (available from W.R. Grace Davison), NexSil™ (available from NNTI).

Many silica sols are prepared from sodium silicate and inevitably contain sodium. It is, however, found that the presence of sodium ions can cause sintering of the silica body at high temperature and/or affect catalytic performance. Therefore, if silica sols containing sodium are used, a step of ion exchange may be required in order to reduce or remove sodium. To avoid carrying out ion exchange steps, it is convenient to use silica sols that contain very little or, ideally, no detectable traces of sodium and have a pH value of less than 7. Most preferably, the silica sol used in the process is slightly acidic with or without polymeric stabilizers. Non-limiting examples of silica sols that contain no detectable traces of sodium include Bindzil® 2034DI, Levasil® 200, Nalco 1034A, Ultra-Sol 7H or NexSil™ 20A.

In some cases, silica dispersion prepared with alkylammonium might be useful. Non-limiting examples ,of commercially low sodium silica sols stabilized by ammonia or alkylammonium cations include LUDOX® TMA (available from W.R. Grace Davison) or VP WR 8520 from Evonik.

The silica sols with higher $SiO_2$ content than 30 wt.% and even up to 50 wt.%, for example W1250, W1836, WK341, WK7330 from Evonik are particularly preferred.

The preferred source of silicon is a silica sol or a combination of silica sol with precipitated or fumed silica.

Conversion of the methyl mercaptan with the catalyst composition of the present disclosure

When the catalyst composition is ready, the catalyst is filled in a reactor, which can be a fixed bed, a fluidized bed or another suitable reactor. Preferentially, it can be a fixed-bed tubular reactor.

With preference, the catalyst composition is pre-activated before the step of contacting the feed. The pre-activation is a step of drying/calcination and is performed at high temperature, preferably between 350 and 550°C. The catalyst composition is preferably calcinated for at least 1 hour, preferentially for at least 6 hours. The one or more zeolites are calcinated before the step of contacting in a nitrogen atmosphere. The step of calcination provides for a crystalline structure to the zeolite.

In a preferred embodiment, a diluent can be added in the gaseous feed comprising said one or more methyl mercaptans. Said diluent can be one or more of hydrogen sulphide, steam, C1-C4 alkanes, $CO_2$, $N_2$ or monocyclic aromatics (e.g. benzene, toluene and/or xylene).

With preference, the weight of feed flowing per unit of weight of the catalyst per hour (weight hourly space velocity, WHSV) is comprised between 0.1 $h^{-1}$ and 100 $h^{-1}$, preferentially comprised between 1.0 $h^{-1}$ and 15 $h^{-1}$. More preferably, WHSV is comprised between 1.5 $h^{-1}$ and 10 $h^{-1}$. Even more preferably, WHSV is comprised between 2.0 $h^{-1}$ and 6.0 $h^{-1}$. This means that the catalyst of the present disclosure can convert a weight of the feed that is superior to the amount of the catalyst present in the reactor.

For example, step c) is performed at a temperature ranging from 200°C to 600°C; preferably ranging from 250°C to 550°C, more preferably, ranging from 300°C to 500°C; even more preferably from 320 °C to 480°C; most preferably to 350 °C to 450°C; and even most preferably from 370°C to 420°C.

For example, step c) is performed at a temperature of at most 600°C; preferably, of at most 550°C; more preferably, of at most 500°C; even more preferably, of at most 480°C; most preferably, of at most 450°C and even most preferably, of at most 420°C.

For example, step c) is performed at a temperature of at least 200°C; preferably, of at least 250°C; more preferably, of at least 300°C; even more preferably, of at least 320°C; most preferably, of at least 350°C and even most preferably, of at least 370°C.

For example, step c) is performed with a partial pressure of the dimethyl sulphide ranging between 10 kPa and 500 kPa, preferentially between 50 kPa and 200 kPa.

The pressure is ranging from 0.2 and 20.0 MPa, preferably ranging from 0.2 and 10.0 MPa, preferably between 0.3 and 5.0 MPa, even more preferably between 0.5 and 2.0 MPa.

In an embodiment, the step c) of contacting the feed stream with the catalyst composition is followed by a step d) of recovering an effluent comprising C2-C3 olefins, hydrogen sulphide, unreacted sulphur containing compounds, alkane and higher hydrocarbons and optionally said diluent; wherein the selectivity to C2-C3 of at least 50 %; with preference, of at least 70 %.

In an embodiment, the step c) of contacting the feed stream with the catalyst composition is followed by a step d) of recovering an effluent comprising C3 olefins; wherein the selectivity to C3 of at least 40 %; with preference, of at least 45 %.

A separation step can be foreseen in order to isolate the different components of the effluent. For example, the separation step can be performed by distillation.

The composition of the product obtained after the reaction

The products obtained after step c) encompass C2-C3 olefins. Preferably, those olefins are mono-olefins that have from 2 to 3 carbon atoms. Said olefins are ethylene and propylene. Other possible olefins are butylenes, preferably but-1-ene, cis- or trans- but-2-ene, or isobutylene, pentenes preferably pent-1-ene, or pent-2-ene.

In a prefered embodiment, said C2-C3 olefins are present in said stream d) at a concentration of at least 10 mol %, preferably at least 25 mol % based on the total molar percent of the stream recovered at the exit of said step c).

In a most preferred embodiment, ethylene and propylene are present in said stream d) at a concentration of at least 10 mol %, preferably at least 20 mol % based on the total molar percent of the stream recovered at the exit of said step c).

In a preferred embodiment, the yield of C2-C3 olefins at step c) is of at least 50 %, preferably 60 % even more preferably at least 70 %.

The content of aromatics in the stream d) is also a relevant factor. In particular, the molar percent of aromatics in the stream recovered at the exit of said step c) is less than 10 mol %, preferably less than 6 mol %.

In a most prefered embodiment, the yield of aromatics at step c) is less than 15 % preferably less than 10%.

The products of step c) further comprise methyl mercaptan. Preferably, methyl mercaptan is present in said stream d) at a concentration of less than 50 mol. %, preferably less than 40 mol. %.

**Test and determination methods**

The conversion of the one or more methyl mercaptans ($X_{RSH}$) is determined according to formula (2):

$$X_{RSH} = \frac{[RSH]^i - [RSH]^f}{[RSH]^i} x100 \ (2)$$

wherein $[RSH]^i$ and $[RSH]^f$ are the molar amount of the alkyl mercaptan RSH in the (initial) feed and in the (final) effluent (or product stream) respectively.

The selectivity in methane (C1) is determined according to formula (3):

$$S_{methane} = \frac{[CH_4]}{[CH_4]+2[C_2H_4]+2[C_2H_6]+3[C_3H_6]+3[C_3H_8]+4[C_4H_8]+4[C_4H_{10}]+\cdots} x100 \ (3)$$

wherein the numerator is the carbon adjusted molar amount of methane and the denominator is the sum of all the carbons adjusted molar amount of all hydrocarbons in the effluent.

The selectivity in ethylene (C2=) is determined according to formula (4):

$$S_{ethylene} = \frac{2\,[C_2H_4]}{[CH_4]+2[C_2H_4]+2[C_2H_6]+3[C_3H_6]+3[C_3H_8]+4[C_4H_8]+4[C_4H_{10}]+\cdots} x100 \ (4)$$

wherein the numerator is the carbon adjusted molar amount of ethylene and the denominator is the sum of all the carbons adjusted molar amount of all hydrocarbons in the effluent.

The selectivity in propylene (C3=) is determined according to formula (5):

$$S_{propylene} = \frac{3\,[C_3H_6]}{[CH_4]+2[C_2H_4]+2[C_2H_6]+3[C_3H_6]+3[C_3H_8]+4[C_4H_8]+4[C_4H_{10}]+\cdots} x100 \ (5)$$

wherein the numerator is the carbon adjusted molar amount of propylene and the denominator is the sum of all the carbons adjusted molar amount of all hydrocarbons in the effluent.

Gas chromatography experiments were carried out to determine quantitatively the selectivity of the reaction. It was performed on a silica BOND column (60 m x 0.32 mm) using Agilent GC operated by ChemStation software equipped with FID & FPD detectors.

Inductively coupled plasma (ICP) optical emission spectrometry was used to determine the chemical composition of the zeolites using a Varian ICP-OES 720-ES. The Si/Al molar ratio or the Si/(Al+M) molar ratio are determined using the said method.

<u>Nitrogen adsorption</u> was used to determine the surface area of the zeolite.

The adsorption of a nitrogen gas on zeolite can be quantitatively described by an adsorption isotherm, which represents the amount of condensed molecules (the adsorbates) in a porous zeolite (the absorbent) as a function of the partial pressure of the gas phase at a constant temperature. Before the measurements, zeolites were degassed overnight at 300°C. The Brunauer-Emmett-Teller (BET) surface area was calculated by the ASAP 2020 physisorption analyzer built-in software (Micromeritics Corp.). Surface area of the zeolite was calculated using a BET (Brunauer, Emmett, and Teller 1938) single-point method from the measured amount of $N_2$ uptake at liquid nitrogen temperature.

<u>Scanning Electron Microscopy</u> was used to determine the size and shape of the zeolite.

The crystal morphology was studied using a Jeol JS 6701F field emission scanning electron microscope (Jeol Ltd.). The working distance was 8-8.3 mm, the accelerating voltage was 3 kV, probe current was $9.6*10-5$ A, emission current was 10 $\mu$A, extraction voltage was 6.96 kV.

## Examples

### Example 1

A catalyst composition comprising SSZ-13 zeolite of CHA family was prepared using the techniques known in the art (J. Catal. 2013, 298, 27). The as-prepared zeolite had Si/Al atomic ratio of 15 and surface area of 826 $m^2$/g, as determined by nitrogen adsorption. The catalyst was further extruded with $SiO_2$ binder, crushed and seized between 35-45 mesh screens and loaded into the reactor. Before the test run it was pre-activated and pre-sulphidized with $N_2/H_2/H_2S$ mixture 96/2/2 mol.%. The reactor was operated at 0.2 MPa and 400°C. The flow of $CH_4/CH_3SH \sim 2/1$ mol. with WHSV of 1 $h^{-1}$ was used. The zeolite was present in the catalyst composition in a content of about 80 wt.% based on the total weight of the catalyst composition.

### Example 2

A catalyst composition comprising SSZ-39 zeolite of AEI family was prepared using the techniques known in the art (ACS Catal. 2015, 5, 10, 6078). The as-prepared zeolite had Si/Al ratio of 10 and surface area of 700 $m^2$/g, as determined by nitrogen adsorption. The catalyst was further extruded with $SiO_2$ binder, crushed and seized between 35-45 mesh screens and loaded into the reactor. Before the test run it was pre-activated and pre-sulphidized with $N_2/H_2/H_2S$ mixture 96/2/2 mol.%. The reactor was operated at 0.2 MPa and 400°C. The flow of $CH_4/CH_3SH \sim 2/1$ mol. with WHSV of 2 $h^{-1}$ was used. The zeolite was present in the catalyst composition in a content of about 80 wt.% based on the total weight the catalyst composition.

### Example 3

A catalyst composition comprising SSZ-13 zeolite of CHA family was prepared using the techniques known in the art. The catalyst was further modified with ammonia molybdate solution to introduce Mo atoms into T-sites of the zeolitic framework.

Isomorphous substitution of SSZ-13 zeolite sample was performed through a post-synthetic treatment. The SSZ-13 zeolite was first ion-exchanged with Cu-ions through washing the sample with 1M solution of $Cu(NO_3)_2$, dried, and subjected to the dealumination. Dealumination was performed by treatment with steam at atmospheric pressure using a tube furnace fitted with a gas flow-through system. The temperature of steaming was 800°C at heating rate of 2 °C/min, and the target temperature was kept for 2h. While increasing the temperature of the furnace, $N_2$ gas (10 L/h) was fed to the tube furnace. As soon as the desired temperature was reached, a mixture of water vapor (10 L/h) and nitrogen (20 g/h) was fed to the furnace. The steamed samples were washed three times with a $NH_4Cl$ solution (0.2 M) for 1 h and then treated with aqueous hydrochloric acid (0.1 M) for 2 h at 100 °C with a solid-to-liquid ratio of 1:100.

After the acid treatment, the material was loaded with molybdenum by wet impregnation with $Na_2MoO_4$ (1 mL of molybdate solution 1.5 mg/mL per ~800 mg of zeolite), dried at room temperature and calcined (550 °C, 5 h). After calcination the zeolite was ion-exchanged in a $NH_4Cl$ solution (0.2 M) for 1 h, washed once with water, dried at 80 °C and calcined (550 °C, 5 h).

The final Si/(Al+Mo) ratio was found to be of 14, as defined by ICP analysis. The catalyst was further extruded with $SiO_2$ binder, crushed and seized between 35-45 mesh screens and loaded into the reactor. Before the test run it was pre-activated and pre-sulphidized with $N_2/H_2/H_2S$ mixture 96/2/2 mol.%. The reactor was operated at 0.2 MPa and 380°C. The flow of $CH_4/CH_3SH \sim 2/1$ mol. with WHSV of 1$h^{-1}$ was used. The zeolite was present in the catalyst composition in a content of about 80 wt.% based on the total weight the catalyst composition.

Table 1: Conversion and selectivity results

| | Example 1 | Example 2 | Example 3 | Comparative example 4[1] |
|---|---|---|---|---|
| Catalyst | Cat. 1 | Cat. 2 | Cat. 3 | H-SSZ-13 |
| Temperature (°C) | 400 | 400 | 380 | 400 |
| Pressure | 0.2 MPa | 0.2 MPa | 0.2 MPa | 0 MPa |
| $p(CH_3SH)$, kPa | 100 | 100 | 100 | 6 |
| WHSV ($CH_3SH$, $h^{-1}$) | 0.5 | 0.5 | 0.5 | 0.4 |
| Zeolite Si/Al | 15 | 10 | 14* | 18.7 |
| **Products** | | | | |
| ethane | 2 | 4 | 4 | 2 |
| C3 | - | - | 1 | 13 |
| C5= | 4 | 2 | 3 | - |
| C4= | 8 | 4 | 15 | 9 |
| C3= (propylene) | 54 | 49 | 50 | 32 |
| C2= (ethylene) | 24 | 32 | 27 | 30 |
| **Conversion (1h)** | **90** | **84** | **96** | **50** |
| **TOS for conversion >50%** | **>9h** | **>9h** | **>10h** | **4h** |
| **Yield of C2-C3 olefins** | **70.2** | **68.0** | **73.9** | **35.5** |
| * Si/(Al+Mo)<br>[1] Chem. Commun., 2021,57, 3323 | | | | |

From the results, it can be seen that an improvement of the selectivity to propylene was obtained, as well as an improvement in the yield of C2-C3 and in the time-on-stream (TOS) parameter.

## Claims

1.  Process for converting methyl mercaptan into C2-C3 olefins, said process comprising the following steps:

    a) providing a gaseous feed stream comprising methyl mercaptan and optionally one or more diluents;
    b) providing a catalyst composition;
    c) contacting the gaseous feed stream with the catalyst composition at a temperature ranging from 100°C to 700°C and at a pressure ranging from 0.2 MPa to 20 MPa, to convert at least part of the methyl mercaptan into C2-C3 olefins; and
    d) recovering said C2-C3 olefins and the unconverted methyl mercaptan if any; **characterized in that** said catalyst composition comprises one or more zeolites with a plurality of pores with a shape of an 8-member ring and **in that** the one or more zeolites have a Si/Al atomic ratio ranging from 2 to 17 as determined by inductively coupled plasma optical emission spectrometry.

2.  The process according to claim 1 is **characterized in that** the one or more zeolites have a Si/Al atomic ratio ranging from 5 to 16 as determined by inductively coupled plasma optical emission spectrometry.

3.  The process according to claim 1 or 2 is **characterized in that** the one or more zeolites are selected from the group of AEI, AFX, CHA, DDR, ERI, EAB, GIS, KFI, LEV, LTA, RHO, PAU, MWF and RTH families, and any mixture thereof; preferably, the one or more zeolites are selected from the group of AEI, CHA, DDR, ERI, KFI, and LEV families, and any mixture thereof; more preferably the one or more zeolites are or comprise AEI and/or CHA families.

4.  The process according to any one of claims 1 to 3 is **characterized in that** the one or more zeolites are or comprise SSZ-39 from the AEI family.

5. The process according to any one of claims 1 to 4 is **characterized in that** the one or more zeolites are or comprise SSZ-13 from the CHA family.

6. The process according to any one of claims 1 to 5 is **characterized in that** at least one zeolite contains at least one metal M being a transition metal and/or a post-transition metal with an atomic ratio Si/(Al+M) ranging from 2 to 17 as determined by inductively coupled plasma optical emission spectrometry.

7. The process according to any one of claims 1 to 6 is **characterized in that** at least one zeolite contains at least one metal M being a transition metal and/or a post-transiton metal wherein the transition metal is selected from Fe, W, V, Mo, Zr, Ag, Ni, Cu, Ti, Zn and any mixture thereof and the post-transition metal is selected from Sn and/or In.

8. The process according to any one of claims 1 to 7 is **characterized in that** the one or more zeolites are present in the catalyst composition at a content ranging from 25 wt. % to 90 wt. % based on the total weight of said catalyst composition; preferably ranging from 50 to 90 wt.%.

9. The process according to any one of claims 1 to 8 is **characterized in that** the catalyst composition further comprises a binder, wherein said binder is selected from silica, clays, alumina phosphates, calcium phosphates, magnesium phosphates, mullite and any mixture thereof; with preference, the binder is or comprises silica.

10. The process according to any one of claims 1 to 9 is **characterized in that** the catalyst composition further comprises a binder, wherein said binder is present in an amount of at least 10 wt.% as based on the total weight of the catalyst composition, preferably in an amount of at least 20 wt.%.

11. The process according to any one of claims 1 to 10 is **characterized in that** methyl mercaptan is present in the gaseous feed stream at a concentration of at least 10 vol % based on the total molar content of the gaseous feed stream and/or **in that** the process further comprises a step of recycling a part of the C2-C3 olefins recovered in the gaseous feed stream.

12. The process according to any one of claims 1 to 11 is **characterized in that** the process further comprises a step of activation of the catalyst composition that is performed before said step c) of contacting the gaseous feed stream; and/or **in that** step c) is performed at a temperature ranging from 300°C to 500°C.

13. The process according to any one of the claims 1 to 12 is **characterized in that** step c) is performed in the absence of $H_2$ and/or **in that** the process further comprises a step of recycling the unconverted dimethyl sulfide back into step c).

14. The process according to any one of claims 1 to 13, **characterized in that** the gaseous feed stream comprises one or more diluents, wherein the one or more diluents are selected from hydrogen sulphide, steam, methane, ethane, propane, butane, carbon dioxide, carbon monoxide, nitrogen, and any mixture thereof; and/or **in that** the gaseous feed stream comprises one or more selected from methanethiol; ethanethiol; 1-propanethiol; 2-propanethiol; allyl mercaptan; butanethiol; *tert*-butyl mercaptan; pentanethiols; or any mixture thereof.

15. Use of a catalyst composition in a process for converting dimethyl sulphide into C2-C3 olefins; **characterized in that** the catalyst composition comprises one or more zeolites with a plurality of pores with a shape of an 8-member ring and **in that** the one or more zeolites have a Si/Al atomic ratio ranging from 2 to 17 as determined by inductively coupled plasma optical emission spectrometry; with preference, the process is according to any one of claims 1 to 14.

Figure 1

Figure 2

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | M. YU ET AL.: "Selective methanethiol-to-olefins conversion over HSSZ-13 zeolite", CHEMICAL COMMUNICATIONS, vol. 57, no. 27, 5 March 2021 (2021-03-05), pages 3323-3326, XP002806116, * page 3323, column 1, paragraph 1 – page 3324, column 2, paragraph 2 * * page 3325, column 1 * ----- | 1-15 | INV. C07C1/32 C07C11/04 C07C11/06 B01J29/70 B01J29/78 |

TECHNICAL FIELDS
SEARCHED      (IPC)

C07C

The present search report has been drawn up for all claims

| Place of search | Date of completion of the search | Examiner |
|---|---|---|
| The Hague | 4 April 2022 | Delanghe, Patrick |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
...............................................................................................
& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070278136 A
- US 4543434 A
- US 4822938 A
- US 20030060672 A
- US 10399858 B
- US 4544538 A, S.I. Zones

**Non-patent literature cited in the description**

- **M. YU et al.** *Chem. Commun.,* 2021, vol. 57, 3323
- Atlas of Zeolite Framework Types. Elsevier, 2007
- Compendium of Chemical Terminology. 1997
- *J. Catal.,* 2013, vol. 298, 27
- *ACS Catal.,* 2015, vol. 5 (10), 6078
- *Chem. Commun.,* 2021, vol. 57, 3323